# EUROPEAN PATENT APPLICATION

(11) **EP 4 699 566 A1**
(43) Date of publication of application: **25.02.2026**
(21) Application number: 24806233.3
(22) Date of filing: 09.04.2024
(51) Int. Cl.: A61B 34/37

(54) **SURGICAL ROBOTIC ARM AND SURGICAL ROBOT**

(30) Priority: 15.05.2023 CN 202310545617
(71) Applicant: Ronovo (Shanghai) Medical Science and Technology Ltd., Shanghai 201102 (CN)
(72) Inventor: LI, Qiang, Shanghai 201102 (CN); LI, Ming, Shanghai 201102 (CN); CAO, Anquan, Shanghai 201102 (CN)
(74) Representative: Murgitroyd & Company
(86) International application number: PCT/CN2024/086863
(87) International publication number: WO 2024/234874

(57) **Abstract**

Disclosed in the present application are a surgical robotic arm (10) and a surgical robot (100). In the surgical robotic arm (10) provided by the present application, a first rotating arm (1) is rotatably connected to a second rotating arm (2); a power unit (3) is provided on the first rotating arm (1), and an execution unit (4) is provided on the second rotating arm (2); the first rotating arm (1) is provided with a first transmission assembly (51), and the second rotating arm (2) is provided with a second transmission assembly (52); the driving force of the power unit (3) is transmitted to the second transmission assembly (52) by means of the first transmission assembly (51) and a transmission shaft (6), and then the second transmission assembly (52) drives the execution unit (4) to act; the driving axis of the execution unit (4) is not parallel to the driving axis of the power unit (3).

## Description

The application claims priority to Chinese patent application No. 202310545617.0 filed with China National Intellectual Property Administration on May 15, 2023, which is hereby incorporated by reference in its entirety.

### TECHNICAL FIELD

The present invention relates to the technical field of medical devices, for example, to a surgical robotic arm and a surgical robot.

### BACKGROUND

The surgical robot can assist doctors in performing more precise operations during surgical operations. When using a surgical robot for a surgical operation, a doctor needs to install a surgical instrument on a distal end of a slave robotic arm of the surgical robot according to the need of a current surgery, then the doctor operates a main manipulator of the surgical robot, and a surgical instrument installed on the slave robotic arm performs corresponding operations under the control of the master manipulator.

In a robotic surgery, the doctor needs to operate the main manipulator for a long time, and the flexibility of the main manipulator will directly affect the doctor's operating experience. Usually, an operation may take several hours, and a bulky main manipulator may easily cause fatigue of the doctor and affect the operation effect. Therefore, the controllability of the main manipulator may be significantly improved by reducing an equivalent inertia of multiple joints of the main manipulator.

In the related art, in order to reduce the equivalent inertia of the multiple joints of the main manipulator and improve the controllability of the main manipulator, the motor with a relatively large mass is arranged at the rear, that is, the motor is arranged close to the base, thus the motor is not arranged at any joint of the main manipulator to avoid increasing the weight at the joint. The driving force from the motor arranged at the rear is transmitted through a gear transmission or a cable transmission to drive the main manipulator to operate, but in this transmission manner, numerous components are involved, the structure is complex, and the transmission distance is limited, thus being unsuitable for a long-distance transmission.

### SUMMARY

The invention provides a surgical robotic arm and a surgical robot with a simple structure. While changing a driving force transmission direction and realizing a long-distance driving force transmission, a rotation of a joint of the surgical robotic arm is not affected.

The invention adopts the following technical solutions.

In one aspect, a surgical robotic arm is provided, including:
a first rotary arm, a first end of the first rotary arm being provided with a power unit;
a second rotary arm, a first end of the second rotary arm being rotationally connected to a second end of the first rotary arm, and a second end of the second rotary arm being provided with an execution unit;
a transmission unit including a first transmission assembly arranged on the first rotary arm and a second transmission assembly arranged on the second rotary arm. An input end of the first transmission assembly is drivingly connected to the power unit, an output end of the first transmission assembly is connected to an input end of the second transmission assembly through a drive shaft, the drive shaft is rotatably arranged at a joint of the first rotary arm and the second rotary arm, and an output end of the second transmission assembly is drivingly connected to the execution unit.

Where, a drive axis of the execution unit is not parallel to a drive axis of the power unit.

On the other hand, a surgical robot is provided, including a surgeon console, a display, and the surgical robotic arm described in any one of the above.

### BRIEF DESCRIPTION OF THE DRAWINGS

FIG. 1 is a schematic structural view of a surgical robotic arm according to an embodiment of the present invention;
FIG. 2 is a schematic structural view of a transmission unit according to an embodiment of the present invention;
FIG. 3 is a schematic view showing an internal structure of the surgical robotic arm according to an embodiment of the present invention;
FIG. 4 is a schematic view showing a partial internal structure of the surgical robotic arm according to an embodiment of the present invention;
FIG. 5 is a schematic structural view of the transmission unit according to another embodiment of the present invention;
FIG. 6 is a schematic view showing a first working state of a second transmission assembly according to an embodiment of the present invention;
FIG. 7 is a schematic view showing a second working state of the second transmission assembly according to an embodiment of the present invention;
FIG. 8 is a schematic structural view of the transmission unit according to another embodiment of the present invention;
FIG. 9 is a schematic view showing a third working state of the second transmission assembly according to an embodiment of the present invention;
FIG. 10 is a schematic structural view showing a first transmission assembly with a primary reduction mechanism according to an embodiment of the present invention;
FIG. 11 is a schematic structural view of the first transmission assembly and the second transmission assembly which are provided with a primary reduction mechanism and a secondary reduction mechanism respectively according to an embodiment of the present invention;
FIG. 12 is a schematic structural view of a surgical robot according to an embodiment of the present invention.

In the drawings:
1. first rotary arm; 2. second rotary arm; 3. power unit; 4. execution unit; 5. transmission unit; 6. drive shaft; 7. primary tensioning mechanism; 8. cross roller bearing;
11. first accommodating cavity; 12. protrusion; 13. first connection seat; 14. first bearing;
21. second accommodating cavity; 22. annular plate; 23. second connection seat; 24. second bearing;
41. execution output shaft; 42. third bearing;
51. first transmission assembly; 511. primary drive pulley; 512. primary driven pulley; 513. primary synchronous belt; 52. second transmission assembly; 521. secondary drive pulley; 522. secondary driven pulley; 523. secondary idler pulley; 524. secondary adjustment pulley; 525. secondary synchronous belt; 526. secondary tensioning mechanism;
71. support bracket; 72. fixed base; 73. adjustment bolt;
100. surgical robot; 10. surgical robotic arm; 20. surgeon console; 30. display.

### DETAILED DESCRIPTION OF THE EMBODIMENTS

In the description of the application, unless otherwise clearly specified and limited, the terms "connect", "communicate", or "fix" should be understood in a broad sense. For example, it may indicate a fixed connection, a detachable connection, or an integral connection; it may indicate a mechanical connection or an electrical connection; it may indicate a direct connection or an indirect connection through an intermediate medium; it may indicate an internal communication of two elements or an interaction relationship between two elements. For the ordinary skilled in the art, the specific meanings of the above terms in this application may be understood according to specific circumstances.

In the present application, unless otherwise clearly specified and limited, for a first feature being "above" or "below" a second feature, it may indicate that the first and second features are in direct contact, or may indicate that the first and second features are not in direct contact but are in contact through another feature therebetween. Moreover, for the first feature being "on", "above", or "over" the second feature, it may indicate that the first feature is directly above and obliquely above the second feature, or simply indicates that the first feature is higher in level than the second feature. For the first feature being "below", "under" or "beneath" the second feature, it may indicate that the first feature is directly below and obliquely below the second feature, or simply indicates that the first feature is lower in level than the second feature.

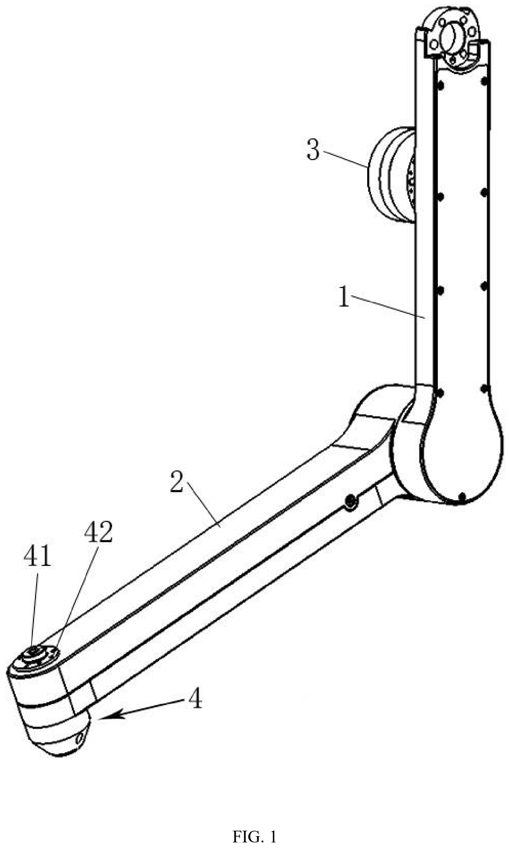

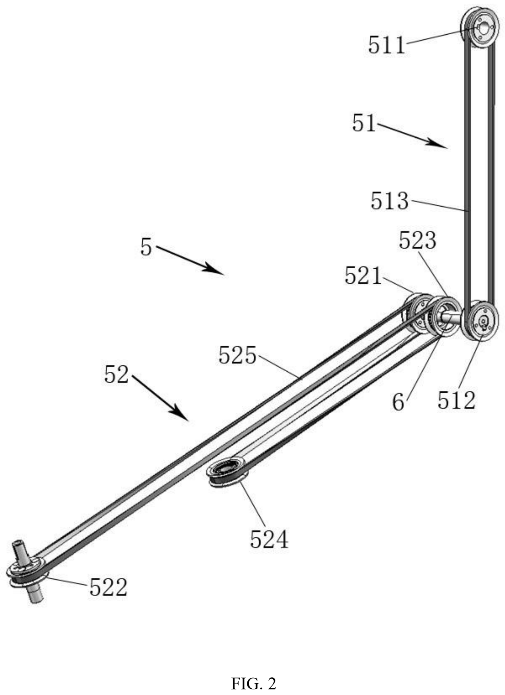

In an embodiment, a surgical robotic arm 10 is provided. In a surgical robot 100, the surgical robotic arm 10 is pivotally connected to a base. As shown in FIG. 1 and FIG. 2, the surgical robotic arm 10 includes a first rotary arm 1, a second rotary arm 2 and a transmission unit 5. A power unit 3 is arranged at a first end of the first rotary arm 1. A first end of the second rotary arm 2 is rotatably connected to a second end of the first rotary arm 1. An execution unit 4 is arranged at a second end of the second rotary arm 2. The transmission unit 5 includes a first transmission assembly 51 arranged on the first rotary arm 1 and a second transmission assembly 52 arranged on the second rotary arm 2. An input end of the first transmission assembly 51 is drivingly connected to the power unit 3, and an output end of the first transmission assembly 51 is connected to an input end of the second transmission assembly 52 through a drive shaft 6. The drive shaft 6 is arranged at a joint of the first rotary arm 1 and the second rotary arm 2. The output end of the second transmission assembly 52 is drivingly connected to the execution unit 4. The drive axis of the execution unit 4 is not parallel to the drive axis of the power unit 3.

For example, the drive axis of the execution unit 4 is perpendicular to the drive axis of the power unit 3, which improves a transmission efficiency of the surgical robotic arm 10.

In the embodiment of the surgical robotic arm 10, the first rotary arm 1 is rotatably connected to the second rotary arm 2, the power unit 3 is arranged on the first rotary arm 1, the execution unit 4 is arranged on the second rotary arm 2, the first rotary arm 1 is provided with the first transmission assembly 51, and the second rotary arm 2 is provided with the second transmission assembly 52, thus the driving force from the power unit 3 is transmitted to the second transmission assembly 52 through the first transmission assembly 51 and the drive shaft 6, and then the second transmission assembly 52 drives the execution unit 4 to operate. The power unit 3 is arranged away from the execution unit 4, thereby reducing the equivalent inertia at a rotation joint of the surgical robotic arm 10, and improving the manipulability of the surgical robotic arm 10, and a relative rotation of the first rotary arm 1 and the second rotary arm 2 is not affected. The drive axis of the execution unit 4 is not parallel to the drive axis of the power unit 3, that is, the first transmission assembly 51 and the second transmission assembly 52 change a driving direction of the power unit 3 to adapt to an operation of the surgical robotic arm 10. The transmission structure can also be adaptable for a long-distance driving force transmission and has a simple structure.

In some other embodiments, the surgical robotic arm 10 may further include a third rotary arm, a fourth rotary arm, etc. The additionally added rotary arms are arranged between the first rotary arm 1 and the second rotary arm 2, and are rotatably connected to the first rotary arm 1 and the second rotary arm 2, respectively. The quantity of rotary arms arranged between the first rotary arm 1 and the second rotary arm 2 is not limited, so as to realize the configuration of multiple rotary joints of the surgical robotic arm 10. Corresponding to the rotary arms, another first transmission assembly 51 or another second transmission assembly 52 is required to be arranged between the aforementioned first transmission assembly 51 and second transmission assembly 52, and are connected by a shaft, so as to realize the transmission of the driving force from the power unit 3 to the execution unit 4.

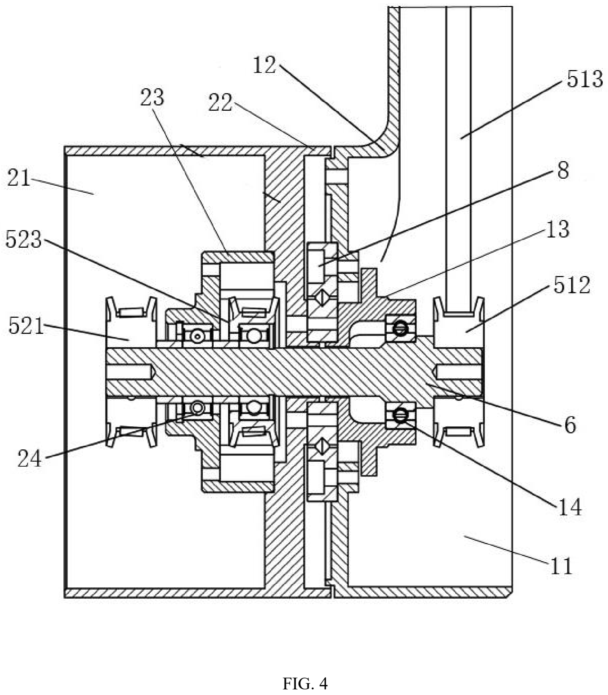

In some embodiments, as shown in FIGS. 2 to 4, the first rotary arm 1 has a first accommodating cavity 11, and the first transmission assembly 51 is disposed in the first accommodating cavity 11. The power unit 3 is disposed on an outer side of the first rotary arm 1. The second rotary arm 2 has a second accommodating cavity 21, and the second transmission assembly 52 is disposed in the second accommodating cavity 21. The execution end of the execution unit 4 is disposed on an outer side of the second rotary arm 2.

The second end of the first rotary arm 1 is connected to the first end of the second rotary arm 2 through a cross roller bearing 8, and the first rotary arm 1 and the second rotary arm 2 are rotatable relative to each other, and the first rotary arm 1 and the second rotary arm 2 are enabled to withstand a relatively large overturning torque. For example, a protrusion 12 is arranged on and protrudes from a side of the second end of the first rotary arm 1, and an annular plate 22 is arranged on and protrudes from a side of the first end of the second rotary arm 2. The annular plate 22 and the second rotary arm 2 enclose to form an accommodating space, and part of the protrusion 12 of the first rotary arm 1 is disposed in the accommodating space, so that the connection between the first rotary arm 1 and the second rotary arm 2 is more compact. The cross roller bearing 8 is arranged in the accommodating space, and one of the second rotary arm 2 and the protrusion 12 is connected to the inner ring of the cross roller bearing 8, and the other thereof is connected to the outer ring of the cross roller bearing 8. The first end of the drive shaft 6 connecting the first transmission assembly 51 and the second transmission assembly 52 is disposed in the first accommodating cavity 11, and a first connection seat 13 is arranged in the first accommodating cavity 11, and the drive shaft 6 is rotatably connected to the first connection seat 13 through a first bearing 14. The second end of the drive shaft 6 passes through the accommodating space and is disposed in the second accommodating cavity 21, and a second connection seat 23 is arranged in the second accommodating cavity 21, and the drive shaft 6 is rotatably connected to the second connection seat 23 through a second bearing 24. The drive shaft 6 is rotatable relative to the first rotary arm 1 and the second rotary arm 2, and while drivingly connecting the first transmission assembly 51 and the second transmission assembly 52, the drive shaft 6 will not affect the rotation of the first rotary arm 1 and the second rotary arm 2. The rotation of the first rotary arm 1 and the second rotary arm 2 and the driving force transmission of the first transmission assembly 51 and the second transmission assembly 52 can be carried out simultaneously without affecting each other.

In some embodiments, the drive axis of the execution unit 4 is not parallel to the drive axis of the power unit 3, for example, the drive axis of the execution unit 4 is perpendicular to the drive axis of the power unit 3. In order to adapt to the connection between the first rotary arm 1 and the second rotary arm 2 and reduce the inertia of the surgical robotic arm, the power unit 3 needs to be provided on the first rotary arm 1, and in this case, the driving force transmission needs to adapt to the rotatable connection between the first rotary arm 1 and the second rotary arm 2. In some embodiments, the drive axis of the execution unit 4 is perpendicular to or not parallel to the drive axis of the drive shaft 6, while the drive axis of the power unit 3 is parallel to the drive axis of the drive shaft 6, so it can be obtained that the drive axis of the execution unit 4 is perpendicular to or not parallel to the axis of the power unit 3.

As shown in FIG. 1, the execution unit 4 includes an execution output shaft 41, and the execution output shaft 41 is connected to the output end of the second transmission assembly 51. One end of the execution output shaft 41 is disposed on the outer side of the second rotary arm 2 and adapted to drive the execution unit 4 to rotate. In order to improve the rotation stability of the execution output shaft 41, the execution output shaft 41 is connected to the second rotary arm 2 through a third bearing 42.

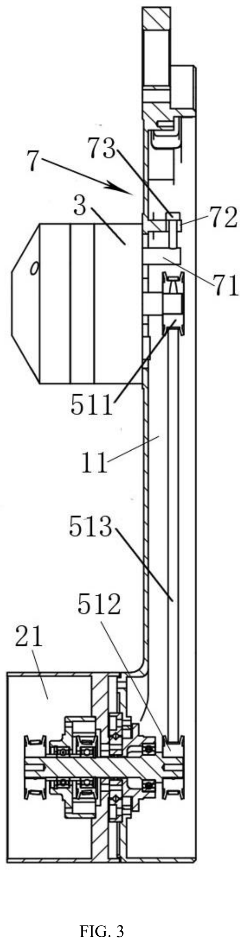

Continuing to refer to FIGS. 2 and 3, the power unit 3 includes a driving motor, and the output shaft of the driving motor is drivingly connected to the input end of the first transmission assembly 51.

In some embodiments, the first transmission assembly 51 includes a primary drive pulley 511, a primary driven pulley 512, and a primary synchronous belt 513. The primary drive pulley 511 is drivingly connected to the power unit 3, for example, the driving motor is drivingly connected to the primary drive pulley 511. The primary driven pulley 512 is connected to the first end of the drive shaft 6, and the primary synchronous belt 513 is wrapped around the primary drive pulley 511 and the primary driven pulley 512. The transmission structure has a simple structure and a good transmission stability.

When the first transmission assembly 51 has run for a long time, the primary synchronous belt 513 may become loose. In order to prevent the primary synchronous belt 513 from skipping teeth and ensure the effectiveness of the transmission of the primary synchronous belt 513, a primary tensioning mechanism 7 is arranged and adapted to adjust the tightness of the primary synchronous belt 513. For example, the primary tensioning mechanism 7 is attached to the power unit 3, and the primary tensioning mechanism 7 is arranged on the first rotary arm 1, and the primary tensioning mechanism 7 can adjust the position of the power unit 3 along the transmission direction of the primary synchronous belt 513. The position of the power unit 3 can be adjusted by adjusting the primary tensioning mechanism 7, as the primary drive pulley 511 is connected to the power unit 3, the position of the primary drive pulley 511 is adjusted, thereby reducing or increasing the distance between the primary drive pulley 511 and the primary driven pulley 512, and achieving the purpose of adjusting the tightness of the primary synchronous belt 513.

For example, the primary tensioning mechanism 7 includes a support bracket 71, a first end of the support bracket 71 is disposed in the first accommodating cavity 11, and a second end of the support bracket 71 is adapted to pass through a side of the first rotary arm 1 and connected to the power unit 3. The support bracket 71 is movable along the transmission direction of the primary synchronous belt 513. A fixed base 72 is disposed on an inner side wall of the first accommodating cavity 11, and an adjustment bolt 73 is screwed to the fixed base 72. The adjustment bolt 73 is adapted to pass through the fixed base 72 and connected to the support bracket 71. The support bracket 71 can be driven to move by screwing the adjustment bolt 73, thereby driving the power unit 3 to move, adjusting the position of the primary drive pulley 511, reducing or increasing the distance between the primary drive pulley 511 and the primary driven pulley 512, and achieving the purpose of adjusting the tightness of the primary synchronous belt 513.

In some other embodiments, the power unit 3 is disposed in the first accommodating cavity 11 and connected to the support bracket 71, which provides a protection for the power unit 3 to some degree.

In some other embodiments, the primary tensioning mechanism 7 includes a primary tensioning pulley, and the primary tensioning pulley is adapted to abut against an outer side or inner side of the primary synchronous belt 513, and the tightness of the primary synchronous belt 513 is changed by adjusting the position of the primary tensioning pulley.

In some embodiments, the secondary transmission assembly 52 includes a secondary drive pulley 521, a secondary driven pulley 522, a secondary idler pulley 523, a secondary adjustment pulley 524, and a secondary synchronous belt 525. The secondary drive pulley 521 is connected to the second end of the drive shaft 6, and the secondary driven pulley 522 is drivingly connected to the execution unit 4. The secondary idler pulley 523 is rotatably disposed on the drive shaft 6, and the secondary idler pulley 523 does not rotate along with the drive shaft 6. The secondary adjustment pulley 524 is rotatably disposed on the second rotary arm 2. The secondary synchronous belt 525 is sequentially wrapped around the secondary drive pulley 521, the secondary driven pulley 522, the secondary idler pulley 523, and the secondary adjustment pulley 524. The tightness of the secondary synchronous belt 525 can be adjusted by adjusting the position of the secondary adjustment pulley 524. The adjustment mechanism connected to the secondary adjustment pulley 524 is a related art and is not be described in detail here. The second transmission assembly 52 has a simple structure and is also provided with the tensioning structure for the secondary synchronous belt 525, thereby effectively avoiding tooth skipping and a problem of invalid transmission of the second transmission assembly 52 due to the loosened secondary synchronous belt 525.

The secondary drive pulley 521 and the secondary idler pulley 523 are coaxially arranged, and the rotational axis of the secondary driven pulley 522 and the rotational axis of the secondary drive pulley 521 are not parallel. For example, the rotational axis of the secondary driven pulley 522 and the rotational axis of the secondary drive pulley 521 are perpendicular to each other, so as to improve the transmission efficiency and realize the change of the driving force transmission direction. The rotational axis of the secondary adjustment pulley 524 is coplanar with the rotational axis of the secondary driven pulley 522. Or the rotational axis of the secondary adjustment pulley 524 is parallel to the rotational axis of the secondary drive pulley 522. Or the rotational axis of the secondary adjustment pulley 524 is located between the rotational axis of the secondary driven pulley 522 and the rotational axis of the secondary drive pulley 521. In an embodiment, the rotational axis of the secondary adjustment pulley 524 is parallel to the rotational axis of the secondary driven pulley 522, and the tensioning structure connected to the secondary adjustment pulley 524 may be arranged on a side of the second rotary arm 2 to facilitate an operation and an adjustment.

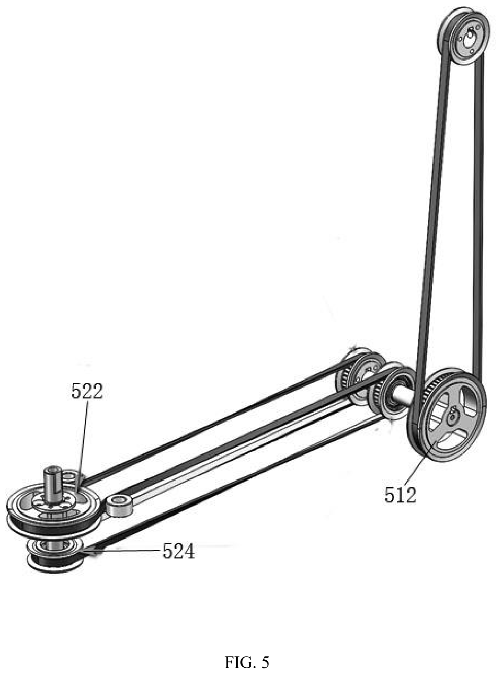

In another embodiment, as shown in FIG. 5, the secondary adjustment pulley 524 and the secondary driven pulley 522 are coaxially arranged, and the positions of the secondary adjustment pulley 524 and the secondary driven pulley 522 can be adjusted simultaneously to adjust the tightness of the secondary synchronous belt 525, thereby simplifying the structure, and reducing the quantity of the configured shafts.

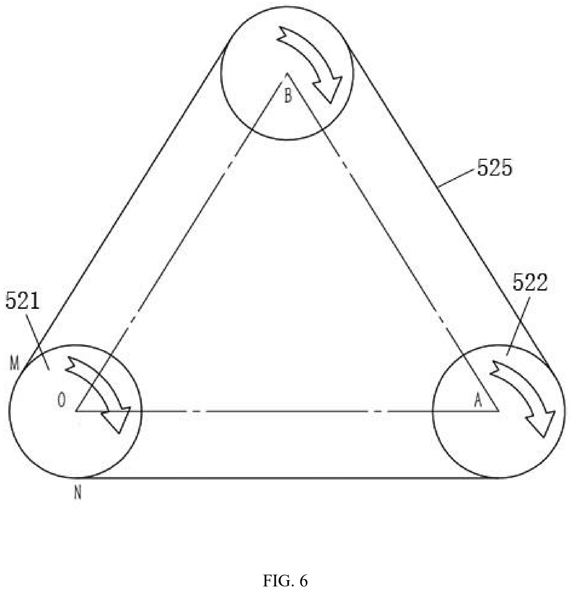

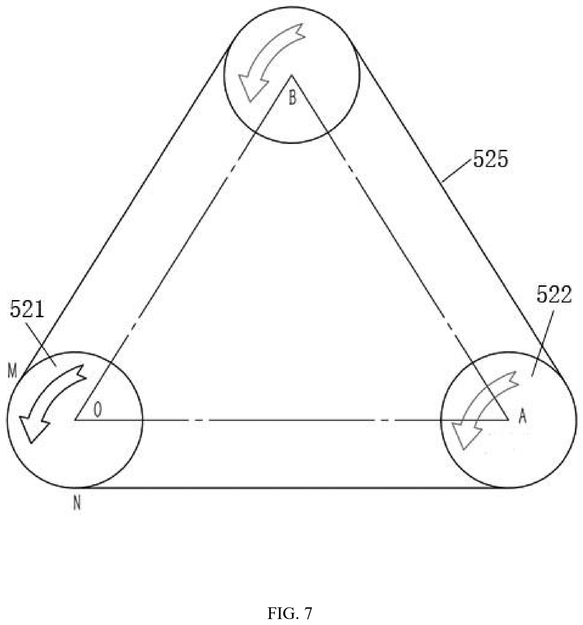

Based on the structure of the second transmission assembly 52, the case where the secondary synchronous belt 525 may skip teeth is different from the case where a conventional synchronous belt skips teeth. For a conventional synchronous belt, when the load is too large, a belt tooth climbs to the top of a pulley tooth and instantly passes over the pulley tooth and moves to an adjacent tooth groove while the belt is moving from a loose side to a tight side. In the second transmission assembly 52 of this embodiment, the secondary synchronous belt 525 may also skip teeth when the design load is not exceeded for the following reasons. Referring to FIGS. 6 and 7, the secondary idler pulley 523 and the secondary adjustment pulley 524 may be regarded as the same idler pulley. In the case where the secondary driven pulley 522 is fixed (i.e., blocked from rotating), when the secondary drive pulley 521 rotates clockwise, as shown in FIG. 6, a side OA of the secondary synchronous belt 525 is a tight side and can transmit torques normally; and when the secondary drive pulley 521 rotates counterclockwise, as shown in FIG. 7, a side OB and a side AB are tight sides, and the side OA is a loose side, and in this case, since the tight sides are too long, the tight sides elongate greatly under the action of a tension, and the secondary drive pulley 521 will rotate to a certain degree, thus causing the loose side OA to become looser. At this time, a point N in the loose side begins to be separated from the secondary drive pulley 521. As the torque of the secondary drive pulley 521 increases, the elongation of the tight sides increases, and the separation state spreads from the point N to the point M, eventually causing tooth skipping.

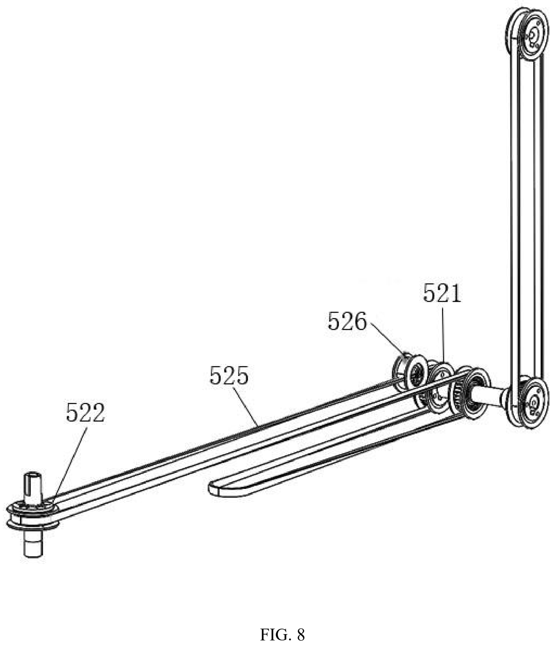

As shown in FIG. 8, the secondary transmission assembly 52 also includes a secondary tensioning mechanism 526, and the secondary tensioning mechanism 526 includes a secondary tensioning pulley. The secondary tensioning pulley is adapted to abut against the secondary synchronous belt 525 between the secondary drive pulley 521 and the secondary driven pulley 522, and the secondary tensioning pulley is arranged to be proximate to the secondary drive pulley 521. The tightness of the secondary synchronous belt 525 may be adjusted by adjusting the position of the secondary tensioning pulley, thereby preventing tooth skipping.

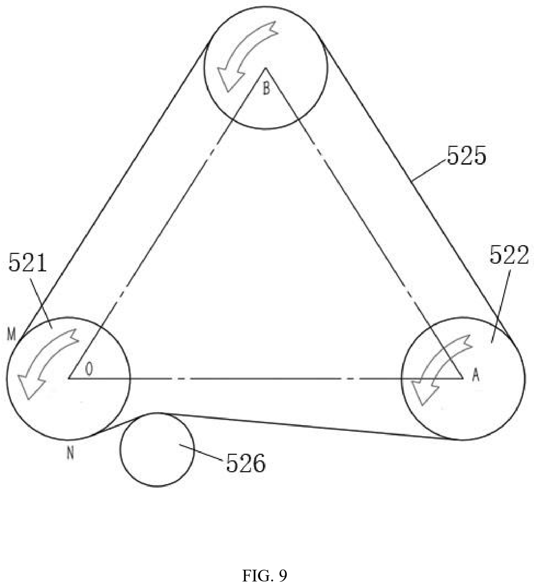

As shown in FIG. 9, after analysis, it is obtained that when the secondary tensioning pulley is arranged on the side OA and close to the point N, a larger wrapped angle can be formed and the space of the tooth skipping points can be compressed under the condition of not increasing the belt length of the secondary synchronous belt 525 as much as possible. Therefore, the secondary tensioning pulley may be arranged at the point N to prevent the secondary synchronous belt 525 at the point N from being separated from the secondary drive pulley 521, thereby significantly reducing the occurrence of the tooth skipping of the secondary synchronous belt 525 and increasing the transmittable torque.

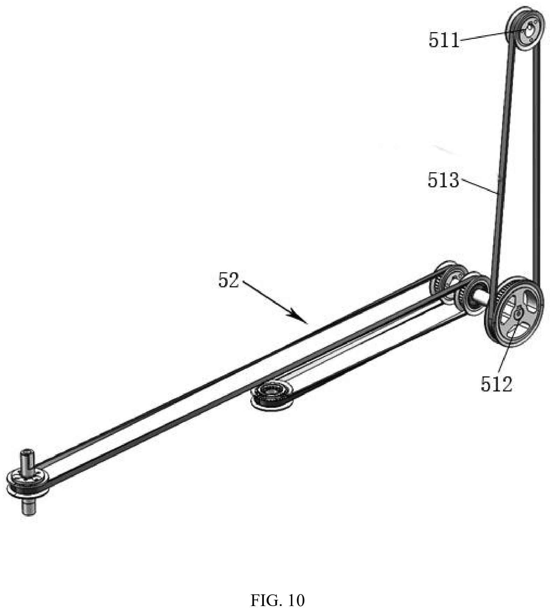

In some embodiments, as shown in FIG. 10, the first transmission assembly 51 is provided with a primary reduction mechanism (including, for example, a primary synchronous belt 513) to reduce the driving power of the power unit 3, thereby reducing the size of the power unit 3 and reducing the entire volume and weight of the surgical robotic arm 10.

Based on the structure of the first transmission assembly 51, in the primary reduction mechanism, increase the quantity of teeth of the primary driven pulley 512 by replacing with a different toothed pulley to achieve an increase in the reduction ratio, therefore the structure is simple and no other additional components are needed.

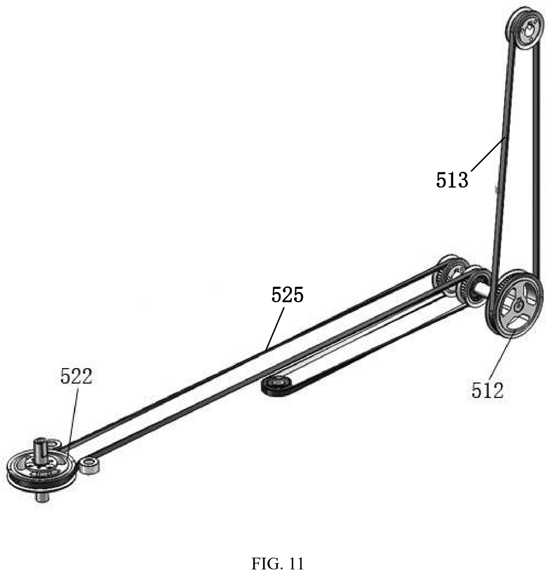

In other embodiments, as shown in FIG. 11, the second transmission assembly 52 is provided with a secondary reduction mechanism (including, for example, a secondary synchronous belt 525) to reduce the driving power of the power unit 3, thereby reducing the size of the power unit 3, and reducing the entire volume and weight of the surgical robotic arm 10.

Based on the structure of the second transmission assembly 52, in the secondary reduction mechanism, increase the quantity of teeth of the secondary driven pulley 522 by replacing with a different toothed pulley to achieve an increase in the reduction ratio, therefore the structure is simple and no other additional components are needed.

In other embodiments, as shown in FIG. 11, the primary reduction mechanism (including, for example, the primary synchronous belt 513) is disposed on the first transmission assembly 51, and the secondary reduction mechanism (including, for example, the secondary synchronous belt 525) is disposed on the second transmission assembly 52 to further increase the reduction ratio and reduce the driving power of the power unit 3, thereby reducing the size of the power unit 3 and reducing the entire volume and weight of the surgical robotic arm 10.

For example, the primary reduction mechanism increases the quantity of the teeth of the primary driven pulley 512, and the secondary reduction mechanism increases the quantity of the teeth of the secondary driven pulley 522, thereby increasing the reduction ratio, and the structure is simple.

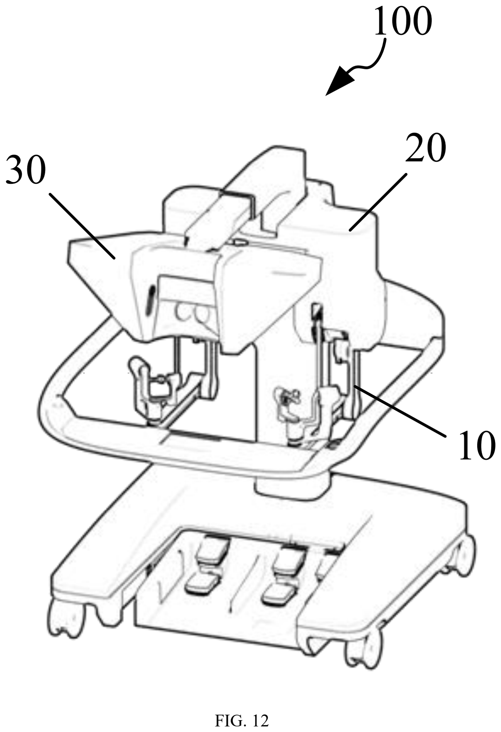

As shown in FIG. 12, an embodiment further provides a surgical robot 100, including a surgeon console 20, a display 30, and the surgical robotic arm 10 as described above.

The surgical robot 100 may be a remote-operated surgical robot, and the surgical robotic arm 10 is a main manipulator. In some other embodiments, the surgical robot 100 may also be any other type of robot, and the surgical robotic arm 10 may be any other robotic arm with a driving force transmission function, which are not specifically limited herein.

## Claims

1. A surgical robotic arm (10), **characterized by** comprising:
a first rotary arm (1), a first end of the first rotary arm (1) being provided with a power unit (3);
a second rotary arm (2), a first end of the second rotary arm (2) being rotationally connected to a second end of the first rotary arm (1), and a second end of the second rotary arm (2) being provided with an execution unit (4); and
a transmission unit (5) comprising a first transmission assembly (51) arranged on the first rotary arm (1) and a second transmission assembly (52) arranged on the second rotary arm (2), wherein an input end of the first transmission assembly (51) is drivingly connected to the power unit (3), an output end of the first transmission assembly (51) is connected to an input end of the second transmission assembly (52) through a drive shaft (6), the drive shaft (6) is rotatably arranged at a joint of the first rotary arm (1) and the second rotary arm (2), and an output end of the second transmission assembly (52) is drivingly connected to the execution unit (4);
wherein, a drive axis of the execution unit (4) is not parallel to a drive axis of the power unit (3).

2. The surgical robotic arm according to claim 1, wherein the first transmission assembly (51) comprises:
a primary drive pulley (511) drivingly connected to the power unit (3);
a primary driven pulley (512) connected to a first end of the drive shaft (6); and
a primary synchronous belt (513) wrapped around the primary drive pulley (511) and the primary driven pulley (512).

3. The surgical robotic arm according to claim 2, wherein a primary tensioning mechanism (7) is attached to the power unit (3), the primary tensioning mechanism (7) is arranged on the first rotary arm (1), and the primary tensioning mechanism (7) is adapted to adjust a position of the power unit (3) along a transmission direction of the primary synchronous belt (513).

4. The surgical robotic arm according to claim 1, wherein the second transmission assembly (52) comprises:
a secondary drive pulley (521) connected to a second end of the drive shaft (6);
a secondary driven pulley (522) drivingly connected to the execution unit (4);
a secondary idler pulley (523) rotatably disposed on the drive shaft (6);
a secondary adjustment pulley (524) rotatably disposed on the second rotary arm (2); and
a secondary synchronous belt (525), sequentially wrapped around the secondary drive pulley (521), the secondary driven pulley (522), the secondary idler pulley (523) and the secondary adjustment pulley (524).

5. The surgical robotic arm according to claim 4, wherein a rotational axis of the secondary adjustment pulley (524) is coplanar with a rotational axis of the secondary driven pulley (522); or
the secondary adjustment pulley (524) and the secondary driven pulley (522) are coaxially arranged.

6. The surgical robotic arm according to claim 5, wherein, when the rotational axis of the secondary adjustment pulley (524) is coplanar with the rotational axis of the secondary driven pulley (522), the rotational axis of the secondary adjustment pulley (524) is parallel to the rotational axis of the secondary driven pulley (522).

7. The surgical robotic arm according to claim 4, wherein the secondary transmission assembly (52) further comprises a secondary tensioning mechanism (526); the secondary tensioning mechanism (526) comprises a secondary tensioning pulley; and the secondary tensioning pulley is adapted to abut against the secondary synchronous belt (525) between the secondary drive pulley (521) and the secondary driven pulley (522), and the secondary tensioning pulley is arranged to be proximate to the secondary drive pulley (521).

8. The surgical robotic arm according to claim 1, wherein the axis of the drive shaft (6) is parallel to the drive axis of the power unit (3), and the drive axis of the execution unit (4) is perpendicular to the drive axis of the power unit (3).

9. A surgical robot (100), comprising a surgeon console (20), a display (30), and the surgical robotic arm (10) according to any one of claims 1 to 8.
